# EUROPEAN PATENT SPECIFICATION

(11) **EP 4 103 187 B1**
(45) Date of publication and mention of the grant of the patent: **06.08.2025**
(21) Application number: 21753591.3
(22) Date of filing: 08.01.2021
(51) Int. Cl.: A61K 31/551, A61P 25/28, A61K 45/06, A61K 31/506, A61K 31/519, A61K 39/395

(54) **FASUDIL FOR TREATING PSEUDOBULBAR AFFECT**
FASUDIL ZUR BEHANDLUNG DES PSEUDOBULBÄREN AFFEKTS
FASUDIL POUR TRAITER UN AFFECT PSEUDO-BULBAIRE

(30) Priority: 10.02.2020 US 202062972440 P; 08.04.2020 US 202063007221 P
(43) Date of publication of application: 21.12.2022
(73) Proprietor: Woolsey Pharmaceuticals, Inc., St. Petersburg, FL 33702 (US)
(72) Inventor: MACALLISTER, Thomas, Arlington, Virginia 22201 (US); JACOBSON, Sven, New York, New York 10005 (US)
(74) Representative: Canet, Denis
(86) International application number: PCT/US2021/012577
(87) International publication number: WO 2021/162808

(56) References cited:
- WO-A1-2005/117896
- JP-A- H06 293 643
- US-A1- 2006 205 822
- US-A1- 2012 010 196
- US-A1- 2016 082 015
- US-A1- 2017 002 317
- KAMEI S ET AL: "Effect of fasudil hydrochloride on wandering symptoms of cerebrovascular dementia patients", NEUROLOGICAL THERAPEUTICS = SHINKEI CHIRYO, JP, vol. 13, no. 1, 1 January 1996 (1996-01-01), pages 43 - 50, XP009551805, ISSN: 0916-8443
- KAMEI S ET AL: "EVALUATION OF FASUDIL HYDROCHLORIDE TREATMENT FOR WANDERING SYMPTOMS IN CEREBROVASCULAR DEMENTIA WITH 31P-MAGNETIC RESONANCE SPECTROSCOPY AND XE-COMPUTED TOMOGRAPHY", CLINICAL NEUROPHARMACOLOGY, RAVEN PRESS, NEW YORK, NY, US, vol. 19, no. 5, 1 January 1996 (1996-01-01), pages 428 - 438, XP008046435, ISSN: 0362-5664
- NABIZADEH FARDIN ET AL: "Pseudobulbar affect in neurodegenerative diseases: A systematic review and meta-analysis", JOURNAL OF CLINICAL NEUROSCIENCE, CHURCHILL LIVINGSTONE, GB, vol. 100, 15 April 2022 (2022-04-15), pages 100 - 107, XP087046778, ISSN: 0967-5868, [retrieved on 20220415], DOI: 10.1016/J.JOCN.2022.04.009
- WOODARD TODD J., KIM CHARLES: "Review of the Diagnosis and Management of Pseudobulbar Affect", U.S. PHARMACIST, 17 November 2017 (2017-11-17), XP055849382, Retrieved from the Internet <URL:https://www.uspharmacist.com/article/review-of-the-diagnosis-and-management-of-pseudobulbar-affect>

## Description

This application claims priority to U.S. Provisional application no. 62/972,440, filed on February 10, 2020, and U.S. Provisional application no. 63/007,221, filed April 8, 2020.

### Background of the Invention

Pseudobulbar affect (PBA), also known as emotional incontinence, is an emotional disturbance characterized by uncontrollable episodes of crying and/or laughing, or other emotional displays. PBA occurs secondary to a number of different neurological disorders. Emotions may be exaggerated in that patients may cry uncontrollably at something that is only moderately sad. Emotions may also be mood-incongruent, perhaps laughing when angry, and may be fluid, switching between emotional states, such as crying turning into laughter.

PBA occurs secondary to neurological disease or brain injury and is thought to result from disruptions of neural networks that control the generation and regulation of motor output of emotions. PBA is most commonly observed in people with neurologic injuries such as traumatic brain injury (TBI) and stroke, and neurologic diseases such as dementias including Alzheimer's disease, attention deficit/hyperactivity disorder (ADHD), multiple sclerosis (MS), amyotrophic lateral sclerosis (ALS), pediatric autoimmune neuropsychiatric disorders associated with streptococcal infections (PANDAS), and Parkinson's disease (PD). It has been reported as a symptom of hyperthyroidism, Graves' Disease, or hypothyroidism in combination with depression.

PBA has also been observed in association with a variety of other brain disorders, including brain tumors, Wilson's disease, syphilitic pseudobulbar palsy, and various encephalitides. Rarer conditions associated with PBA include gelastic epilepsy, dacrystic epilepsy, central pontine myelinolysis, olivopontinocerebellar atrophy, lipid storage diseases, chemical exposure (e.g., nitrous oxide and insecticides), fou rire prodromique, and Angelman syndrome.

Kamei (1996) reported on using fasudil in two patients with sub-cortical vascular dementia. The primary outcome was that fasudil mitigated wandering by these patients. One patient was diagnosed with Binswanger-type cerebral infarction, confirmed by MRI imaging. The other patient was diagnosed with sequelae of cerebral bleeding and multiple lacunar infarctions, confirmed by MRI. Kamei indicates that compulsive laughing and/or crying was observed in the patients. Kamei filed a patent application in Japan (Patent Application 6-293643) based on the same two patients in the publication and a third patient. The patients of Kamei were variously reported to exhibit compulsive laughing and/or crying and reported improvements in "emotional incontinence," there was no assessment or diagnosis of PBA, which can be readily confused with depression and other neurological conditions. Furthermore, the results of Kamei are confounded by the fact that it was an open-label report of case studies, it lacked any control group, and the presence of concomitant medications, such as pentoxifylline and vinpocetine, both of which are known to have CNS effects.

### Summary of the Invention

Note that the references to the methods of treatment by therapy of this description are to be interpreted as references to compounds, pharmaceutical compositions and medicaments of the present invention for use in those methods.

One embodiment of the invention involves a method of treating a patient with PBA, preferably having an CNS-LS score of at least 13, comprising treating said patient with a therapeutically effective amount of the rho kinase inhibitor fasudil, hydroxyfasudil, or a pharmaceutically acceptable salt thereof. In certain aspects of this embodiment, the patient is suffering from a condition selected from the group consisting of neurological diseases, traumatic brain injury, stroke, dementia, attention deficit/hyperactivity disorder multiple sclerosis, amyotrophic lateral sclerosis, PANDAS, Parkinson's disease, hyperthyroidism, Graves' Disease, hypothyroidism, a brain tumor, Wilson's disease, syphilitic pseudobulbar palsy, encephalitis, gelastic epilepsy, dacrystic epilepsy, central pontine myelinolysis, olivopontinocerebellar atrophy, a lipid storage disease, chemical exposure, fou rire prodromique, and Angelman syndrome.

A preferred aspect of the invention contemplates a method of treating PBA in a patient suffering from Alzheimer's Disease, frontotemporal dementia, or cortical vascular dementia.

Preferred daily doses range from about 70 mg to about 140 mg per day, with 90 mg per day being particularly preferred. Oral administration is preferred.

In one specific embodiment, the PBA patient is female.

In another specific embodiment, the PBA patient is female having secondary progressive multiple sclerosis.

In one embodiment, the PBA patient does not have subcortical vascular dementia.

In still another embodiment the PBA patient does not have amyotrophic lateral sclerosis (ALS) and the patient may not have subcortical vascular dementia or ALS.

In another embodiment, the PBA patient does not have sleep disturbances or loss of appetite or symptoms of depression.

In a specific embodiment, the patient exhibits a neurological defect affecting the frontal lobe of the brain.

In another embodiment, the PBA patient does not have another neurological or psychiatric disorder.

In a further embodiment, the PBA is a side effect of a drug administered for a neurological or psychiatric disorder.

In another embodiment, treatment with fasudil eliminates the need to treat the PBA patient with dextromethorphan containing drug.

In a further specific embodiment, the PBA patient is a child with ADD or ADHD.

In certain other embodiments, the patient is not concurrently treated with drugs that enhance cerebral blood flow, such as pentoxifylline and vinpocetine.

Preferred embodiments involve treating patient for at least 21 days and generally for at least 30 days.

### Detailed Description of the Invention

The invention is based on the discovery that rho kinase inhibitors can be used to treat PBA.

### ROCK Inhibitors

The inventive methods contemplate the administration of a rho kinase (ROCK) inhibitor in the treatment of a disease or condition. Two mammalian ROCK homologs are known, ROCK1 (aka ROKβ, Rho-kinase β, or p160ROCK) and ROCK2 (aka ROKα) (Nakagawa 1996). In humans, the genes for both ROCK1 and ROCK2 are located on chromosome 18. The two ROCK isoforms share 64% identity in their primary amino acid sequence, whereas the homology in the kinase domain is even higher (92%) (Jacobs 2006; Yamaguchi 2006). Both ROCK isoforms are serine/threonine kinases and have a similar structure.

A large number of pharmacological ROCK inhibitors are known (Feng, LoGrasso, Defert, & Li, 2015). Isoquinoline derivatives are a preferred class of ROCK inhibitors. The isoquinoline derivative fasudil was the first small molecule ROCK inhibitor developed by Asahi Chemical Industry (Tokyo, Japan). The characteristic chemical structure of fasudil consists of an isoquinoline ring, connected via a sulphonyl group to a homopiperazine ring. Fasudil is a potent inhibitor of both ROCK isoforms. *In vivo,* fasudil is subjected to hepatic metabolism to its active metabolite hydroxyfasudil (aka, M3). Other examples of isoquinolone derived ROCK inhibitors include dimethylfasudil and ripasudil.

Other non-claimed ROCK inhibitors are based on 4-aminopyridine structures. These were first developed by Yoshitomi Pharmaceutical (Uehata et al., 1997) and are exemplified by Y-27632. Still other non-claimed ROCK inhibitors include indazole, pyrimidine, pyrrolopyridine, pyrazole, benzimidazole, benzothiazole, benzathiophene, benzamide, aminofurazane, quinazoline, and boron derivatives (Feng et al., 2015). Some exemplary ROCK inhibitors are shown below:

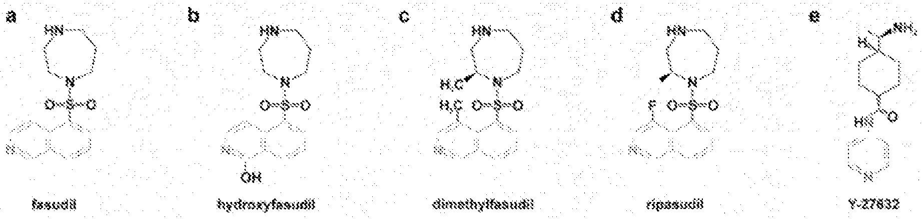

Note that only fasudil and hydroxyfasudil form part of the present invention. ROCK inhibitors according to the invention may have more selective activity for either ROCK1 or ROCK2 and will usually have varying levels of activity on PKA, PKG, PKC, and MLCK. Some ROCK inhibitors may be highly specific for ROCK1 or ROCK2 and have much lower activity against PKA, PKG, PKC, and MLCK.

A particularly preferred ROCK inhibitor is fasudil (hexahydro-1-(5-isoquinolinesulfonyl)-1H-1,4-diazepine). Fasudil may be exist as a free base or salt, preferably the monohydrochloride salt, and may be in the form of a hydrate, such as a hemihydrate. As used herein, it will be understood that methods specifying the active moiety of a ROCK inhibitor apply equally to the free acids or free bases, salts, hydrates, polymorphs thereof.

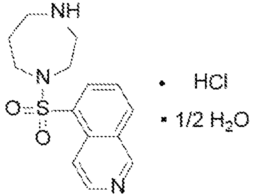

### Hexahydro-1-(5-isoquinolinesulfonyl)-1H-1,4-diazepine monohydrochloride hemihydrate

Fasudil is a selective inhibitor of protein kinases, such as ROCK, PKC and MLCK and treatment results in a potent relaxation of vascular smooth muscle, resulting in enhanced blood flow (Shibuya 2001). A particularly important mediator of vasospasm, ROCK induces vasoconstriction by phosphorylating the myosin-binding subunit of myosin light chain (MLC) phosphatase, thus decreasing MLC phosphatase activity and enhancing vascular smooth muscle contraction. Moreover, there is evidence that fasudil increases endothelial nitric oxide synthase (eNOS) expression by stabilizing eNOS mRNA, which contributes to an increase in the level of the potent vasodilator nitric oxide (NO), thereby enhancing vasodilation (Chen 2013).

Fasudil has a short half-life of about 25 minutes, but it is substantially converted *in vivo* to its 1-hydroxy (M3) metabolite. M3 has similar effects to its fasudil parent molecule, with slightly enhanced activity and a half-life of about 8 hours (Shibuya 2001). Thus, M3 is likely responsible for the bulk of the *in vivo* pharmacological activity of the molecule. M3 exists as two tautomers, depicted below:

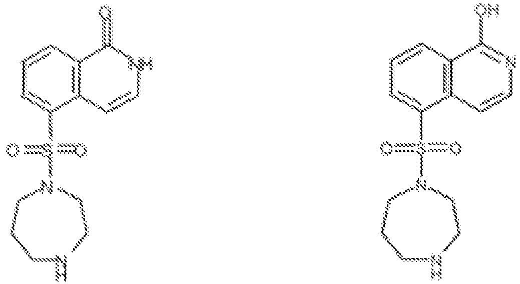

The ROCK inhibitors used in the invention, such as fasudil, include pharmaceutically acceptable salts and hydrates. Salts of fasudil may be formed via reaction with inorganic and organic acid. Those inorganic and organic acids include, but are not limited to, the following: hydrochloric acid, hydrobromide acid, hydriodic acid, sulphuric acid, nitric acid, phosphoric acid, acetic acid, maleic acid, maleic acid, maleic acid, oxalic acid, oxalic acid, tartaric acid, malic acid, mandelic acid, trifluoroacetic acid, pantothenic acid, methane sulfonic acid, or para-toluenesulfonic acid.

### Pharmaceutical Compositions

Pharmaceutical compositions of ROCK inhibitors are oral and may be in the form of tablets or capsules and may be immediate-release formulations (formulations that do not control or retard release of the drug) or may be controlled- or extended-release formulations (such as those described below), which may contain pharmaceutically acceptable excipients, such as corn starch, mannitol, povidone, magnesium stearate, talc, cellulose, methylcellulose, carboxymethylcellulose and similar substances. A pharmaceutical composition comprising a ROCK inhibitor and/or a salt and/or hydrate thereof may comprise one or more pharmaceutically acceptable excipients, which are known in the art. Formulations include oral films, liquids, orally disintegrating tablets, effervescent tablets and granules or beads that can be sprinkled on food or mixed with liquid as a slurry or poured directly into the mouth to be washed down. Pharmaceutical compositions containing ROCK inhibitors, salts and hydrates thereof can be prepared by any method known in the art of pharmaceutics. In general, such preparatory methods include the steps of bringing a ROCK inhibitor or a pharmaceutically acceptable salt or hydrate thereof into association with a carrier or excipient, and/or one or more other accessory ingredients, and then, if necessary and/or desirable, shaping, and/or packaging the product into a desired single- or multi-dose unit.

Pharmaceutical compositions can be prepared, packaged, and/or sold in bulk, as a single unit dose, and/or as a plurality of single unit doses. As used herein, a "unit dose" is a discrete amount of the pharmaceutical composition comprising a predetermined amount of the active ingredient. The amount of the active ingredient is generally equal to the dosage of the active ingredient which would be administered to a subject and/or a convenient fraction of such a dosage such as, for example, one-half or one-third of such a dosage.

Relative amounts of the active ingredient, the pharmaceutically acceptable excipient, and/or any additional ingredients in a pharmaceutical composition of the invention will vary, depending upon the identity, size, and/or condition of the subject treated and further depending upon the route by which the composition is to be administered. The composition used in accordance with the methods of the present invention may comprise between 0.001% and 100% (w/w) active ingredient.

Pharmaceutically acceptable excipients used in the manufacture of provided pharmaceutical compositions include inert diluents, dispersing and/or granulating agents, surface active agents and/or emulsifiers, disintegrating agents, binding agents, preservatives, buffering agents, lubricating agents, and/or oils. Excipients such as coloring agents, coating agents, sweetening, flavoring, and perfuming agents may also be present in the composition.

In certain embodiments, the pharmaceutical composition used in the methods of the present invention may comprise at least one diluent. Exemplary diluents include calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, cornstarch, powdered sugar, and mixtures thereof.

In certain embodiments, the pharmaceutical composition used in the methods of the present invention may comprise at least one granulating and/or dispersing agent. Exemplary granulating and/or dispersing agents include potato starch, corn starch, tapioca starch, sodium starch glycolate, clays, alginic acid, guar gum, citrus pulp, agar, bentonite, cellulose, and wood products, natural sponge, cation-exchange resins, calcium carbonate, silicates, sodium carbonate, cross-linked poly(vinyl-pyrrolidone) (crospovidone), sodium carboxymethyl starch (sodium starch glycolate), carboxymethyl cellulose, cross-linked sodium carboxymethyl cellulose (croscarmellose), methylcellulose, pregelatinized starch (starch 1500), microcrystalline starch, water insoluble starch, calcium carboxymethyl cellulose, magnesium aluminum silicate (VEEGUM), sodium lauryl sulfate, quaternary ammonium compounds, and mixtures thereof.

In certain embodiments, the pharmaceutical composition used in the methods of the present invention may comprise at least one binding agent. Exemplary binding agents include starch (e.g., cornstarch and starch paste), gelatin, sugars (e.g., sucrose, glucose, dextrose, dextrin, molasses, lactose, lactitol, mannitol, etc.), natural and synthetic gums (e.g., acacia, sodium alginate, extract of Irish moss, panwar gum, ghatti gum, mucilage of isapol husks, carboxymethylcellulose, methylcellulose, ethylcellulose, hydroxyethylcellulose, hydroxypropyl cellulose, hydroxypropyl methylcellulose, microcrystalline cellulose, cellulose acetate, poly(vinyl-pyrrolidone), magnesium aluminum silicate (VEEGUM.RTM.), and larch arabogalactan), alginates, polyethylene oxide, polyethylene glycol, inorganic calcium salts, silicic acid, polymethacrylates, waxes, water, alcohol, and/or mixtures thereof.

In certain embodiments, the pharmaceutical composition used in the methods of the present invention may comprise at least one preservative. Exemplary preservatives include antioxidants, chelating agents, antimicrobial preservatives, antifungal preservatives, antiprotozoan preservatives, alcohol preservatives, acidic preservatives, and other preservatives. In certain embodiments, the preservative is an antioxidant. In other embodiments, the preservative is a chelating agent.

In certain embodiments, the pharmaceutical composition used in the methods of the present invention may comprise at least one antioxidant. Exemplary antioxidants include alpha tocopherol, ascorbic acid, acorbyl palmitate, butylated hydroxyanisole, butylated hydroxy toluene, monothioglycerol, potassium metabisulfite, propionic acid, propyl gallate, sodium ascorbate, sodium bisulfite, sodium metabisulfite, and sodium sulfite.

In certain embodiments, the pharmaceutical composition used in the methods of the present invention may comprise at least one chelating agent. Exemplary chelating agents include ethylenediaminetetraacetic acid (EDTA) and salts and hydrates thereof (e.g., sodium edetate, disodium edetate, trisodium edetate, calcium disodium edetate, dipotassium edetate, and the like), citric acid and salts and hydrates thereof (e.g., citric acid monohydrate), fumaric acid and salts and hydrates thereof, malic acid and salts and hydrates thereof, phosphoric acid and salts and hydrates thereof, and tartaric acid and salts and hydrates thereof. Exemplary antimicrobial preservatives include benzalkonium chloride, benzethonium chloride, benzyl alcohol, bronopol, cetrimide, cetylpyridinium chloride, chlorhexidine, chlorobutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, and thimerosal.

In certain embodiments, the pharmaceutical composition may comprise at least one buffering agent together with the ROCK inhibitor or the salt thereof. Exemplary buffering agents include citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, and mixtures thereof.

In certain embodiments, the pharmaceutical composition used in the methods of the present invention may comprise at least one lubricating agent. Exemplary lubricating agents include magnesium stearate, calcium stearate, stearic acid, silica, talc, malt, glyceryl behanate, hydrogenated vegetable oils, polyethylene glycol, sodium benzoate, sodium acetate, sodium chloride, leucine, magnesium lauryl sulfate, sodium lauryl sulfate, and mixtures thereof.

In other embodiments, the pharmaceutical composition containing a ROCK inhibitor or salt or hydrate thereof is administered as a liquid dosage form. Liquid dosage forms for oral administration include pharmaceutically acceptable emulsions, microemulsions, solutions, suspensions, syrups, and elixirs. In addition to the active ingredients, the liquid dosage forms may comprise inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (e.g., cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can include adjuvants such as wetting agents, emulsifying and suspending agents, sweetening, flavoring, and perfuming agents.

Solid dosage forms for oral administration include capsules, tablets, pills, powders, and granules.

In such solid dosage forms, the active ingredient is mixed with at least one inert, pharmaceutically acceptable excipient or carrier such as sodium citrate or dicalcium phosphate and/or (a) fillers or extenders such as starches, lactose, sucrose, glucose, mannitol, and silicic acid, (b) binders such as, for example, carboxymethylcellulose, alginates, gelatin, polyvinylpyrrolidinone, sucrose, and acacia, (c) humectants such as glycerol, (d) disintegrating agents such as agar, calcium carbonate, potato or tapioca starch, alginic acid, certain silicates, and sodium carbonate, (e) solution retarding agents such as paraffin, (f) absorption accelerators such as quaternary ammonium compounds, (g) wetting agents such as, for example, cetyl alcohol and glycerol monostearate, (h) absorbents such as kaolin and bentonite clay, and (i) lubricants such as talc, calcium stearate, magnesium stearate, solid polyethylene glycols, sodium lauryl sulfate, and mixtures thereof. In the case of capsules, tablets, and pills, the dosage form may include a buffering agent.

Some compositions of the invention relate to extended- or controlled-release formulations. These may be, for example, diffusion-controlled products, dissolution-controlled products, erosion products, osmotic pump systems or ionic resin systems. Diffusion-controlled products comprise a water-insoluble polymer which controls the flow of water and the subsequent egress of dissolved drug from the dosage from. Dissolution-controlled products control the rate of dissolution of the drug by using a polymer that slowly solubilizes or by microencapsulation of the drug - using varying thicknesses to control release. Erosion products control release of drug by the erosion rate of a carrier matrix. Osmotic pump systems release a drug based on the constant inflow of water across a semi permeable membrane into a reservoir which contains an osmotic agent. Ion exchange resins can be used to bind drugs such that, when ingested, the release of drug is determined by the ionic environment within the gastrointestinal tract.

### Methods of Treatment

The invention generally involves a method of treating a patient with PBA, comprising treating said patient with a therapeutically effective amount of the rho kinase inhibitor fasudi, hydroxyfasudil or a pharmaceutically acceptable salt thereof. In certain aspects of this embodiment, the patient is suffering from a condition selected from the group consisting of neurological diseases, traumatic brain injury, stroke, dementia, attention deficit/hyperactivity disorder multiple sclerosis, amyotrophic lateral sclerosis, PANDAS, Parkinson's disease, hyperthyroidism, Graves' Disease, hypothyroidism, a brain tumor, Wilson's disease, pseudobulbar palsy, syphilitic pseudobulbar palsy, encephalitis, gelastic epilepsy, dacrystic epilepsy, central pontine myelinolysis, olivopontinocerebellar atrophy, a lipid storage disease, chemical exposure, fou rire prodromique, and Angelman syndrome.

The Center for Neurologic Study - Lability Scale (CNS-LS) (Moore 1997) is the most widely used instrument for diagnosing PBA. It is a self-administered questionnaire with seven questions which are scored by the patient from one to five. In the event that the patient is unable to answer the questions, they can be answered by a caregiver who spends a meaningful amount of time with the patient. A minimum score of 13 on the CNS-LS is diagnostic of PBA. Thus, the invention contemplates treating patients with a score of 13 or higher on the CNS-LS.

Using the following scale, each item is assessed over the immediately preceding week.
1 = Never
2 = Rarely
3 = Occasionally
4 = Frequently
5 = Most of the time

| **Score** | **Questions** |
|---|---|
| | I find that even when I try to control my laughter I am often unable to do so |
| | I find that I am easily overcome by laughter |
| | There are times when I won't be thinking of anything happy or funny at all, but then I'll suddenly be overcome by funny or happy thoughts |
| | Others have told me that I seem to become amused very easily or that I seem to become amused about things that aren't really funny |
| | I find myself crying very easily |
| | There are times when I feel fine one minute, and then I'll become tearful the next over something small or for no reason at all |
| | I find that even when I try to control my crying I am often unable to do so |
| | **Total Score** |

Preferred embodiments contemplate treating PBA in patients with dementia. Dementia broadly results from damage to one of two areas of the brain: the cortex (aka, the cerebral cortex) and the subcortex. The differentiation between cortical (of the cortex) and subcortical (of the subcortex) forms of dementia can often be done by observing the deficits and relating them back to the brain structures associated with that function.

The subcortex consists of three main divisions. The first is the basal ganglia, which is involved in motor control and skills learning. Defects in this area cause either hypokinetic or hyperkinetic problems. Parkinson's and Huntington's disease affect the basal ganglia. The second is the limbic system, which primarily functions in the detection and expression of emotion. It consists of the amygdala, which detects fearful or threatening objects, and the hippocampus, which is involved in laughter. The connection between the amygdala, thalamus (part of the diencephalon) and hippocampus is associated with positive feelings. The hippocampus also plays an important role in learning, memory and detecting novelty. The third is the diencephalon, which consists of the thalamus and hypothalamus. The thalamus is the main sensory relay for all senses, except smell, between the sense organs. The hypothalamus regulates body temperature, hunger, sexual behavior and thirst.

The cerebral cortex is the outermost layer of the cerebrum. It is made up of four lobes and is involved in complex brain functions including memory, attention, perceptual awareness, "thinking," language and consciousness. It also controls voluntary motor function. Thus, the cortex is often described functionally, in terms of its primary sensory and motor areas.

The parietal, temporal and occipital lobes are involved in producing our perceptions resulting from what our eyes see, ears hear and other sensory organs tell us about the position of different parts of our body and relate them to the position of other objects in the environment. The parietal-temporal-occipital complex, especially of the left hemisphere, is responsible for our understanding and use of language. The frontal lobe is involved in planning actions and movement, as well as abstract thought. The limbic area is involved in emotion and memory

The motor areas are located in both hemispheres of the cortex. The primary motor cortex controls executing voluntary movements. The supplementary motor areas and premotor cortex are involved in selecting voluntary movements. The posterior parietal cortex guides voluntary movements in space. The dorsolateral prefrontal cortex is involved in deciding which voluntary movements to make according to higher-order instructions, rules and self-generated thoughts.

The following table illustrates some broad differences between defects found in cortical versus subcortical forms of dementia.

| **Cortical** | **Subcortical** |
|---|---|
| General lack of motor symptoms | Motor symptoms common |
| No coordination defect | Lack of coordination |
| Normal cognitive processing, wrong answers | Slow, but correct answers |
| Severe amnesia, recall and recognition affected | Better recognition, improved by clues |
| Unable to calculate | Able to calculate |
| Executive abilities preserved | Executive abilities disproportionately affected |
| Aphasia prominent | Normal with dysarthria and less word output |
| Personality intact until late | Apathetic and inert |

Examples of cortical dementia are Alzheimer's (AD), Vascular Dementia, Lewy Body (LBD), Frontotemporal Lobar (FTD; Pick's Disease), Frontotemporal Lobar (FTD; Primary Progressive Aphasia (PPA))
Examples of subcortical dementia are Binswanger's disease (BD; lacunar dementia), Parkinson's Disease (PD), Huntington's Disease (HD) and Multiple Sclerosis (MS).

In accordance with the treatment methods of the present disclosure, an effective amount of a ROCK inhibitor or a pharmaceutically acceptable salt and/or hydrate thereof for administration one or more times a day may comprise from about 10 mg to about 1000 mg. Fasudil hydrochloride hemihydrate, for example, is suitably administered in a daily amount of about 10 mg to about 500 mg, about 50 mg to about 400 mg, about 70 mg to about 200 mg, about 80 mg to about 140 mg, about 90 mg to about 120 mg. One preferred dosing regimen involves treatment with 35 or 40 mg of Fasudil hydrochloride hemihydrate three times per day using an immediate-release formulation, for a total daily dose of 70 - 120 mg. According to the present invention, dosing exceeds a daily dose of 60 mg, with most preferred ranges for daily dosing being 70 mg to 120 mg administered in three equal amounts during the day. A particularly preferred daily dose is 90 mg per day. A further dosing regimen involves treatment with, 35 to 60 mg of Fasudil hydrochloride hemihydrate only two times per day using an immediate-release formulation, for a total daily dose of 70 - 120 mg. A preferred embodiment is 45 mg of fasudil hydrochloride hemihydrate two times per day using an immediate-release formulation.

Certain patient sub-populations, such as renally impaired patients and/or older patients (e.g., 65 or older) may need lower doses or extended release formulations instead of immediate release formulations. Fasudil hydrochloride hemihydrate may have higher steady-state concentrations when given at usual doses to patients with renal disease and lower doses to lower the Cmax or delay the time to Cmax (increase the Tmax) may be required.

Renal dysfunction occurs with age and as the result of numerous disorders, including liver cirrhosis, chronic kidney disease, acute kidney injury (for example, due to administering a contrast agent), diabetes (Type 1 or Type 2), autoimmune diseases (such as lupus and IgA nephropathy), genetic diseases (such as polycystic kidney disease), nephrotic syndrome, urinary tract problems (from conditions such as enlarged prostate, kidney stones and some cancers), heart attack, illegal drug use and drug abuse, ischemic kidney conditions, urinary tract problems, high blood pressure, glomerulonephritis, interstitial nephritis, vesicoureteral, pyelonephritis, sepsis. Kidney dysfunction may occur in other diseases and syndromes, including non-kidney-related diseases that may occur along with kidney dysfunction, for example pulmonary artery hypertension, heart failure, and cardiomyopathies, among others.

Kidney function is most often assessed using serum (and/or urine) creatinine. Creatinine is a breakdown product of creatine phosphate in muscle cells and it is produced at a constant rate. It is excreted by the kidneys unchanged, principally through glomerular filtration. Accordingly, elevated serum creatinine is a marker for kidney dysfunction and it is used to estimate glomerular filtration rate.

Normal levels of creatinine in the blood are approximately 0.6 to 1.2 mg/dL in adult males and 0.5 to 1.1 mg/dL in adult females. When creatinine levels exceed these figures, the subject has renal dysfunction, and is, therefore, treatable according to the invention. Mild renal impairment/dysfunction occurs in the range of 1.2 mg/dL to 1.5 mg/dL. Moderate renal impairment/dysfunction is considered to occur at creatinine levels exceeding 1.5 mg/dL. Severe renal impairment, which includes what is considered to be renal failure, is defined as a serum creatinine level of ≥ 2.0 mg/dL or the use of renal replacement therapy (such as dialysis). Treating subjects with mild, moderate and severe renal impairment is specifically contemplated.

As indicated, creatinine levels are considered to be a surrogate for glomerular filtration rate and serum creatinine levels alone may be used to estimate glomerular filtration rate using the Cockroft-Gault equation.

Generally, creatinine clearance of less than 60 mL/min (corresponding roughly to creatinine of > 1.2 mg/dL) is considered moderate renal dysfunction. A glomerular filtration rate below 40 mL/min (corresponding approximately to creatinine levels exceeding 1.5 mg/dL) or especially 30 mL/min is considered severe renal dysfunction.

In general, creatinine clearance (estimated glomerular filtration rate) may be derived directly from serum creatinine using the Cockroft - Gault equation: creatinine clearance = (((140 - age in years) x (wt in kg)) x 1.23) / (serum creatinine in mmol/L)

For women the result of the calculation is multiplied by 0.85.

Empirically measured creatinine clearance may also be used directly as an estimate of glomerular filtration rate by looking at serum creatinine and urine creatinine levels. Specifically, urine is collected over 24 hours and the following equation is applied to ascertain creatinine clearance: Creatinine Clearance (mL/min) = Urine Creatinine Concentration (mg/mL) * 24 hour urine volume (mL)/Plasma Creatinine Concentration (mg/mL) * 24 hour * 60 minutes

In one embodiment, dose of fasudil for mild to moderate renal impairment is reduced to 50-80 mg per day. In another embodiment, the dose of fasudil is not reduced but is administered one time per day in an extended release dosage form.

In another embodiment, the dose is not reduced for mild to moderate renal impairment.

In one embodiment, the dose of fasudil is reduced to 30-45 for severe renal impairment. In another embodiment, the dose of fasudil is not reduced but is instead administered one time per day in an extended release dosage form.

In a further embodiment, the dose is reduced where serum creatinine (SCr) >2 and/or an increase in SCr > 1.5x from baseline, and/or a decrease in eGFR >25% from baseline.

Patient size is an important factor to consider when using creatinine-based estimates of renal function. The units of drug clearance are volume/time (mL/min), whereas the units of estimated GFR for chronic renal disease are volume/time/**standard size** (mL/min/1.73m²). Generally, doses may be adjusted down (e.g., 40-50 mg per day) for smaller patients and up for larger (e.g., 120 mg per day) for obese patients. A smaller male would be about 160 pounds or less. A smaller female patient would weigh about 130 pounds or less. Patients having a Body Mass Index of 30 and higher is considered obese.

In addition, older patients may need a lower dose at initiation, with a gradual increase to the recommended dose after days or weeks. In another embodiment, older patients may need lower doses for the duration of treatment. The aged population includes the "young old" who are 65-74, the "old old" who are 75-84 and the "frail elderly" who are 85 and older. For example, a starting dose of 30 mg per day for two weeks, followed by 60 mg per day for 4 weeks, then by 90 mg per day. Titration may even be warranted up to about 120 mg per day.

Another embodiment involves the treatment with 60-120 mg of fasudil hydrochloride hemihydrate once per day in an extended release dosage form. Treatment with an extended release total daily dose of 90 mg fasudil hydrochloride hemihydrate once per day is preferred. It will be appreciated that dose ranges as described herein provide guidance for the administration of provided pharmaceutical compositions to an adult. The amount to be administered to, for example, a child or an adolescent can be determined by a medical practitioner or person skilled in the art and can be lower or the same as that administered to an adult.

It will be appreciated that dose ranges as described herein provide guidance for the administration of provided pharmaceutical compositions to an adult. The amount to be administered to, for example, a child or an adolescent can be determined by a medical practitioner or person skilled in the art and can be lower or the same as that administered to an adult. It will also be appreciated by the skilled person that all dosing regimens may be converted using a standard human weight of 70 kg to daily doses expressed in milligrams per kilogram. Thus, a preferred daily dosing range according to the invention would be 1 - 2 mg/kg, with a particularly preferred daily dose being 1.3 mg/kg.

Methods of administering compositions according to the invention would generally be continued for at least one day. Some preferred methods treat daily for up to 30 days or daily up to 60 days or even daily up to 90 days or even more. Daily treatment for more than 60 days is preferred and daily treatment for at least 6 months is particularly preferred. The precise duration of treatment will depend on the patient's condition and response to treatment.

Patients treatable according to the invention will typically score poorly on cognitive scales, such as the mini mental state exam (MMSE). A threshold of ≤ 23 on the MMSE is set for dementia, with score of ≤15 Representing severe dementia. Once the MMSE falls below 15, the Severe Impairment Battery (SIB) is a useful assessment too. Thus, the invention particularly contemplates treating patients with an MMSE score ≤ 23, including moderately demented patients having an MMSE score of 16-23 and severe patients having an MMSE score ≤ 15. Generally, once a patient has an MMSE score of less than 9, they may develop problems walking and treatment of patients with an MMSE less than 5 is not preferred. Treatment using the inventive methods generally result in improved cognitive functioning. Patients will generally show improvement on the MMSE and the SIB of at least 3 points during the early stages of treatment and declines in cognition are slowed relative to control patients.

Also contemplated is a method of administering the ROCK inhibitors, preferably fasudil, to patients having a patient-reported Center for Neurologic Study-Lability Scale (CNS-LS) score of 13 or greater, 14 or greater, 15 or greater, 16 or greater, 17 or greater, 18 or greater, 19 or greater, or 20 or greater. The CNS-LS is a patient-administered questionnaire designed to provide a quantitative measure of the patient's perceived frequency of episodes correlated with PBA.

Particularly preferred methods involve treating patients suffering from PBA secondary to Alzheimer's Disease and Traumatic Brain Injury. Treating PBA secondary to large vessel ischemic stroke is also preferred.

The methods of the invention also contemplate administering the ROCK inhibitors, preferably fasudil, with other compounds used to treat dementia or other symptoms of dementia. They may be administered in combination, as a single dosage form, in a common dosing regimen, or administered to the same patient at different times of the day using different dosing regimens. In one embodiment, treatment of a PBA patient with fasudil reduces the frequency of episodes and/or intensity of emotional episodes. In specific embodiments, the frequency of episodes is reduced by about 10%, about 15%, about 20%, about 30%, about 40%, or about 50% or greater. In one embodiment, the episodes are measured daily. In another embodiment, the episodes are measure weekly. In yet another embodiment the episodes are measured monthly.

In another embodiment, treatment of a PBA patient with fasudil reduces the number of situationally inappropriate episodes, e.g., inappropriate laughing where the context is not appropriate for laughter. In specific embodiments, the frequency of situationally inappropriate episodes is reduced by about 10%, about 15%, about 20%, about 30%, about 40%, or about 50% or greater. In one embodiment, the episodes are measured daily. In another embodiment, the episodes are measure weekly. In yet another embodiment the episodes are measured monthly. In various embodiments of the invention, the patient experiences reductions in PBA about 10%, about 15%, about 20%, about 30%, about 40%, or about 50% or greater as measured by one or more of the following scales, all of which will be known to one of ordinary skill in the art: the CNS-LS; the Pathological Laughter and Crying Scale (PLACS); the Clinical Global Impression of Severity of Illness (CGIS) Scale the Clinical Global Impression of Change (CGIC) Scale; the Patient Global Impression of Change (PGIC) Scale; the Neuropsychiatric Inventory-Nursing Home (NPI-NH) Questionnaire; the Impact of Pseudobulbar Affect (PBA) on Participant Scale; the Minimum Data Set (MDS) Sections of Presumed Relevance to PBA, Including Sections on Speech, Cognition, Mood, Behavior, Health Condition, and Medication; a decrease in the use of Concomitant Psychotropic Medication; and the Impact of PBA on Informant Scale (see, for example: ClinicalTrials.gov Identifier: NCT02496039).

In another embodiment, treatment of a PBA patient with fasudil reduces the CNS-LS score by at least one point, preferably by at least two points, and more preferably by three points or more. In a specific embodiment, treatment of a PBA patient with fasudil reduces the CNS-LS score to below 17.

In some embodiments, the patients are administered fasudil in combination with other actives approved to treat cortical dementia, including but not limited to cholinesterase inhibitors and N-methyl-D-aspartate (NMDA) receptor antagonists. In one embodiment, the cholinesterase inhibitor is selected from the group consisting of donepezil, rivastigmine, and galantamine. Exemplary doses of the cholinesterase inhibitors include 3-25 mg per day, more preferably 6-12 mg per day. In another embodiment, the NMDA receptor antagonist is memantine. In a specific embodiment, memantine is administered at a dose of 5-28 mg per day, preferably 15-20 mg per day. In a further embodiment, the co-administered active is a combination of donepezil and memantine at a dose of 28 mg memantine and 10 mg donepezil.

Dextromethorphan hydrobromide is another an uncompetitive NMDA receptor antagonist that also has activity as a sigma-1 receptor agonist. Marketed in combination quinidine sulfate (a CYP450 2D6 inhibitor), the product NUEDEXTA^{®} is indicated for the treatment of pseudobulbar affect, which occurs in many forms of dementia. In a specific embodiment, NUEDEXTA^{®} is co-administered in a dose of 10-30 mg/day, preferably 20 mg/day. In one embodiment, the combination with fasudil is administered with lower doses of NUEDEXTA^{®}. In another embodiment, fasudil is administered without (i.e., instead of) NUEDEXTA^{®}.

In some embodiments, the inventive methods contemplate administering the ROCK inhibitors in combination with or in patients being treated with antidepressants. Suitable antidepressants, including tricyclic antidepressants, selective serotonin reuptake inhibitors and norepinephrine/serotonin reuptake inhibitors. Tricyclic antidepressants useful according to the invention include amitriptyline and nortriptyline. Selective serotonin reuptake inhibitors include citalopram, fluoxetine, sertraline, fluvoxamine, escitalopram and paroxetine. Norepinephrine/serotonin reuptake inhibitors include venlafaxine, duloxetine, sibutramine, desvenlafaxine, milnacipran, levomilnacipran, and tomoxetine.

Lower doses of antidepressants are used than the doses required to treat depression when used in combination with the ROCK inhibitors. Generally, doses of antidepressants used in combinations with the

ROCK inhibitors will be lower than the lowest doses recommended in the official prescribing information (www.fda.gov). In most cases, antidepressant doses will be no more than 20% of the lowest recommended dose. In certain cases, doses will be no more than 10% or even 5% of the lowest recommended dose.

In certain embodiments, patients taking antidepressants for PBA will no longer require antidepressants to control their symptoms. Thus, methods are contemplated wherein patients are treated with the rho kinase inhibitors, but are not treated with antidepressants in addition to the rho kinase inhibitors.

In other embodiments, combinations with agents including antipsychotics and mood stabilizers is contemplated. Antipsychotics include atypical antipsychotics (e.g., olanzapine, clozapine, quetiapine, risperidone, aripiprazole); phenothiazine antipsychotics (e.g., prochlorperzine, fluphenazine, chlorpromazine, trifluoperazine, thioridazine, perphenazine); and thiothixene. Mood stabilizers include lithium, carbamazepine, lamotrigine, valproate, and asenapine. Combinations with other agents including benzodiazepines (e.g., lorazepam diazepam, estazolam, alprazolam, alprazolam, quazepam, chlorazepate, clonazepam, flurazepam, oxazepam) is also contemplated.

### LIST OF REFERENCES

Chen M, Liu A, Ouyang Y, Huang Y, Chao X, Pi R. 2013. Fasudil and its analogs: a new powerful weapon in the long war against central nervous system disorders? Expert Opin Investig Drugs. 22:537-50.
Feng Y, LoGrasso P, Defert O, Li R, Rho Kinase (ROCK) Inhibitors and Their Therapeutic Potential. J Med Chem. 2016; 59*6): 2269-2300.
Jacobs M, Hayakawa K, Swenson L, Bellon S, Fleming M, Taslimi P, Doran J, The structure of dimeric ROCK I reveals the mechanism for ligand selectivity. J Biol Chem. 2006; 281(1): 260-68.
Kamei S, Toshiaki T, Oishi M, Effect of fasudil hydrochloride on wandering symptoms of c cerebrovascular dementia patients. Neurotherapy. 1996b 13:43-50.
Moore SR, Gresham LS, Bromberg MB, Kasarkis EJ, Smith RA. 1997. A self report measure of affective lability. J Neurol Neurosurg Psychiatry. 63:89-93.
Nakagawa O, Fukisawa K, Ishizaki T, Saito Y, Nakao K, Narumiya S, ROCK-I and ROCK-II, two isoforms of Rho-associated coiled-coil forming protein serine/threonine kinase in mice. FEBS Lett. 1996 Aug 26;392(2):189-93.
Nakaoka A, Suto S, Makimoto K, Yamakawa M, Shigenobu K, Tabushi K. 2010.
Pacing and lapping movements among institutionalized patients with dementia. Am J Alzheimers Dis Other Demen. 25:167-72.
Shibuya M, Asano T, Sasaki Y. 2001. Effect of Fasudil HCl, a protein kinase inhibitor, on cerebral vasospasm. Acta Neurochir Suppl. 77:201-4.
Uehata M, Ishizaki T, Satoh H, Ono T, Kawahara T, Morishita T, Tamakawa H, Yamagami K, Maekawa M, Narumiya S, Calcium sensitization of smooth muscle mediated by a Rho-associated protein kinase in hypertension. Nature. 1997 Oct 30;389(6654):990-4.
Yamaguchi H, Miwa Y, Kasa M, Kitano K, Amano M, Kaibuchi K, Hakoshima T, Structural basis for induced-fit binding of Rho-kinase to the inhibitor Y-27632. J Biochem. 2006 Sep;140(3):305-11.

## Claims

1. A pharmacologically effective amount of fasudil, hydroxyfasudil (M3), or a pharmaceutically acceptable salt thereof, for use in the treatment of a patient suffering from pseudobulbar affect, wherein the fasudil or hydroxyfasudil is orally administered and exceeds a daily dose of 60 mg.

2. The pharmacologically effective amount of fasudil, hydroxyfasudil (M3), or a pharmaceutically acceptable salt thereof, for use according to claim 1, wherein the patient is suffering from a condition selected from the group consisting of neurological diseases, traumatic brain injury, stroke, dementia, attention deficit/hyperactivity disorder multiple sclerosis, amyotrophic lateral sclerosis, PANDAS, Parkinson's disease, hyperthyroidism, Graves' Disease, hypothyroidism, a brain tumor, Wilson's disease, syphilitic pseudobulbar palsy, bulbar palsy, encephalitis, gelastic epilepsy, dacrystic epilepsy, central pontine myelinolysis, olivopontinocerebellar atrophy, a lipid storage disease, chemical exposure, fou rire prodromique, and Angelman syndrome.

3. The pharmacologically effective amount of fasudil, hydroxyfasudil (M3), or a pharmaceutically acceptable salt thereof, for use according to claim 2, wherein the patient is suffering from Alzheimer's Disease, frontotemporal dementia, or cortical vascular dementia.

4. The pharmacologically effective amount of fasudil, hydroxyfasudil (M3), or a pharmaceutically acceptable salt thereof, for use according to claim 2, wherein the patient does not have sub-cortical vascular dementia.

5. The pharmacologically effective amount of fasudil, hydroxyfasudil (M3), or a pharmaceutically acceptable salt thereof, for use according to claim 1, wherein the fasudil or hydroxyfasudil is administered at a daily dose of 70 mg to 120 mg in three equal portions throughout the day.

6. The pharmacologically effective amount of fasudil, hydroxyfasudil (M3), or a pharmaceutically acceptable salt thereof, for use according to claim 1, wherein the fasudil or hydroxyfasudil is administered in an extended release formulation.

7. The pharmacologically effective amount of fasudil, hydroxyfasudil (M3), or a pharmaceutically acceptable salt thereof, for use according to claim 6, wherein the fasudil is fasudil hydrochloride hemihydrate administered at daily dose of 90 mg once per day.

8. The pharmacologically effective amount of fasudil, hydroxyfasudil (M3), or a pharmaceutically acceptable salt thereof, for use according to claim 1, wherein the fasudil is fasudil hydrochloride hemihydrate administered 35 to 60 mg two times per day using an immediate-release formulation.

9. The pharmacologically effective amount of fasudil, hydroxyfasudil (M3), or a pharmaceutically acceptable salt thereof, for use according to claim 1, wherein the patient is female.

10. The pharmacologically effective amount of fasudil, hydroxyfasudil (M3), or a pharmaceutically acceptable salt thereof, for use according to claim 1, wherein the patient has Center for Neurologic Study-Lability Scale (CNS-LS) score of 13 or greater.

11. The pharmacologically effective amount of fasudil, hydroxyfasudil (M3), or a pharmaceutically acceptable salt thereof, for use according to claim 1, wherein the fasudil or hydroxyfasudil is administered in a dose of 70 to 140 mg/day.

## Patentansprüche

1. Eine pharmakologisch wirksame Menge Fasudil, Hydroxyfasudil (M3) oder eines pharmazeutisch verträglichen Salzes davon, zur Verwendung bei der Behandlung eines Patienten, der an einer pseudobulbären Affektstörung leidet, wobei das Fasudil oder Hydroxyfasudil oral verabreicht wird und eine Tagesdosis von 60 mg überschreitet.

2. Die pharmakologisch wirksame Menge Menge Fasudil, Hydroxyfasudil (M3) oder eines pharmazeutisch verträglichen Salzes davon, zur Verwendung gemäß Anspruch 1, wobei der Patient an einer Erkrankung leidet, ausgewählt aus der Gruppe bestehend aus neurologischen Erkrankungen, traumatischer Hirnverletzung, Schlaganfall, Demenz, Aufmerksamkeitsdefizit-/Hyperaktivitätsstörung, multipler Sklerose, amyotropher Lateralsklerose, PANDAS, Parkinson-Krankheit, Hyperthyreose, Morbus Basedow, Hypothyreose, Hirntumor, Morbus Wilson, syphilitischer Pseudobulbärparalyse, Bulbärparalyse, Enzephalitis, gelastischer Epilepsie, dacrystischer Epilepsie, zentraler pontiner Myelinolyse, olivopontozerebellärer Atrophie, einer Lipidspeicherkrankheit, chemischer Exposition, Lachtod und Angelman-Syndrom.

3. Die pharmakologisch wirksame Menge Fasudil, Hydroxyfasudil (M3) oder eines pharmazeutisch verträglichen Salzes davon, zur Verwendung gemäß Anspruch 2, wobei der Patient an Alzheimer-Krankheit, frontotemporaler Demenz oder kortikaler vaskulärer Demenz leidet.

4. Die pharmakologisch wirksame Menge Fasudil, Hydroxyfasudil (M3) oder eines pharmazeutisch verträglichen Salzes davon, zur Verwendung gemäß Anspruch 2, wobei der Patient nicht an subkortikaler vaskulärer Demenz leidet.

5. Die pharmakologisch wirksame Menge Fasudil, Hydroxyfasudil (M3) oder eines pharmazeutisch verträglichen Salzes davon, zur Verwendung gemäß Anspruch 1, wobei das Fasudil oder Hydroxyfasudil in einer Tagesdosis von 70 mg bis 120 mg in drei gleichen Portionen über den Tag verteilt verabreicht wird.

6. Die pharmakologisch wirksame Menge Fasudil, Hydroxyfasudil (M3) oder eines pharmazeutisch verträglichen Salzes davon, zur Verwendung gemäß Anspruch 1, wobei das Fasudil oder Hydroxyfasudil in einer Formulierung mit verlängerter Wirkstofffreisetzung verabreicht wird.

7. Die pharmakologisch wirksame Menge Fasudil, Hydroxyfasudil (M3) oder eines pharmazeutisch verträglichen Salzes davon, zur Verwendung gemäß Anspruch 6, wobei das Fasudil Fasudilhydrochlorid-Hemihydrat ist, das einmal täglich in einer Tagesdosis von 90 mg verabreicht wird.

8. Die pharmakologisch wirksame Menge Fasudil, Hydroxyfasudil (M3) oder eines pharmazeutisch verträglichen Salzes davon, zur Verwendung gemäß Anspruch 1, wobei das Fasudil Fasudilhydrochlorid-Hemihydrat ist, das zwei Mal täglich in einer Dosis von 35 bis 60 mg unter Verwendung einer Formulierung mit sofortiger Wirkstofffreisetzung verabreicht wird.

9. Die pharmakologisch wirksame Menge Fasudil, Hydroxyfasudil (M3) oder eines pharmazeutisch verträglichen Salzes davon, zur Verwendung gemäß Anspruch 1, wobei der Patient weiblich ist.

10. Die pharmakologisch wirksame Menge Fasudil, Hydroxyfasudil (M3) oder eines pharmazeutisch verträglichen Salzes davon, zur Verwendung gemäß Anspruch 1, wobei der Patient einen Wert von 13 oder mehr auf der Center for Neurologic Study-Lability-Skala (CNS-LS) aufweist.

11. Die pharmakologisch wirksame Menge Fasudil, Hydroxyfasudil (M3) oder eines pharmazeutisch verträglichen Salzes davon, zur Verwendung gemäß Anspruch 1, wobei das Fasudil oder Hydroxyfasudil in einer Dosierung von 70 bis 140 mg/Tag verabreicht wird.

## Revendications

1. Une quantité pharmacologiquement efficace du fasudil, hydroxyfasudil (M3) ou d'un sel pharmaceutiquement acceptable de ceux-ci, pour l'utilisation dans le traitement d'un patient souffrant d'affect pseudobulbaire, ledit fasudil ou hydroxyfasudil étant administré par voie orale et dépassant une dose journalière de 60 mg.

2. La quantité pharmacologiquement efficace du fasudil, hydroxyfasudil (M3) ou d'un sel pharmaceutiquement acceptable de ceux-ci, pour l'utilisation selon la revendication 1, le patient souffrant d'une affection choisie dans le groupe constitué de maladies neurologiques, traumatisme crânien, accident vasculaire cérébral, démence, trouble du déficit de l'attention avec ou sans hyperactivité, sclérose en plaques, sclérose latérale amyotrophique, PANDAS, maladie de Parkinson, hyperthyroïdie, maladie de Basedow, hypothyroïdie, tumeur cérébrale, maladie de Wilson, paralysie pseudobulbaire syphilitique, paralysie bulbaire, encéphalite, épilepsie gélastique, épilepsie dacrystique, myélinolyse centropontine, atrophie olivopontocérébelleuse, maladie de stockage des lipides, exposition chimique, fou rire prodromique et syndrome d'Angelman.

3. La quantité pharmacologiquement efficace du fasudil, hydroxyfasudil (M3) ou d'un sel pharmaceutiquement acceptable de ceux-ci, pour l'utilisation selon la revendication 2, le patient souffrant de la maladie d'Alzheimer, de démence frontotemporale ou de démence vasculaire corticale.

4. La quantité pharmacologiquement efficace du fasudil, hydroxyfasudil (M3) ou d'un sel pharmaceutiquement acceptable de ceux-ci, pour l'utilisation selon la revendication 2, le patient ne souffrant pas de démence vasculaire souscorticale.

5. La quantité pharmacologiquement efficace du fasudil, hydroxyfasudil (M3) ou d'un sel pharmaceutiquement acceptable de ceux-ci, pour l'utilisation selon la revendication 1, ledit fasudil ou hydroxyfasudil étant administré à une dose quotidienne de 70 mg à 120 mg répartie en trois portions égales au cours de la journée.

6. La quantité pharmacologiquement efficace du fasudil, hydroxyfasudil (M3) ou d'un sel pharmaceutiquement acceptable de ceux-ci, pour l'utilisation selon la revendication 1, ledit fasudil ou hydroxyfasudil étant administré sous forme de formulation à libération prolongée.

7. La quantité pharmacologiquement efficace du fasudil, hydroxyfasudil (M3) ou d'un sel pharmaceutiquement acceptable de ceux-ci, pour l'utilisation selon la revendication 6, ledit fasudil étant le fasudil chlorhydrate hémihydraté administré une fois par jour à une dose quotidienne de 90 mg.

8. La quantité pharmacologiquement efficace du fasudil, hydroxyfasudil (M3) ou d'un sel pharmaceutiquement acceptable de ceux-ci, pour l'utilisation selon la revendication 1, ledit fasudil étant le fasudil chlorhydrate hémihydraté administré deux fois par jour à une dose de 35 à 60 mg sous forme de formulation à libération immédiate.

9. La quantité pharmacologiquement efficace du fasudil, hydroxyfasudil (M3) ou d'un sel pharmaceutiquement acceptable de ceux-ci, pour l'utilisation selon la revendication 1, le patient étant de sexe féminin.

10. La quantité pharmacologiquement efficace du fasudil, hydroxyfasudil (M3) ou d'un sel pharmaceutiquement acceptable de ceux-ci, pour l'utilisation selon la revendication 1, le patient présentant un score de 13 ou plus à l'échelle Center for Neurologic Study-Lability Scale (CNS-LS).

11. La quantité pharmacologiquement efficace du fasudil, hydroxyfasudil (M3) ou d'un sel pharmaceutiquement acceptable de ceux-ci, pour l'utilisation selon la revendication 1, ledit fasudil ou hydroxyfasudil étant administré à une dose de 70 à 140 mg/jour.
